(19) Europäisches Patentamt / European Patent Office / Office européen des brevets

(11) **EP 1 973 582 B1**

(12) **EUROPÄISCHE PATENTSCHRIFT**

(45) Veröffentlichungstag und Bekanntmachung des Hinweises auf die Patenterteilung:
**07.09.2011 Patentblatt 2011/36**

(51) Int Cl.:
*A61L 27/50* (2006.01)          *A61L 27/44* (2006.01)
*A61L 27/14* (2006.01)          *A61F 2/08* (2006.01)
*B29C 61/06* (2006.01)          *A61B 17/04* (2006.01)

(21) Anmeldenummer: 06755020.2

(22) Anmeldetag: **04.05.2006**

(86) Internationale Anmeldenummer:
**PCT/EP2006/062061**

(87) Internationale Veröffentlichungsnummer:
**WO 2006/117398 (09.11.2006 Gazette 2006/45)**

(54) **LANGSAM KONTRAHIERENDES, STEIFES VERBINDUNGSELEMENT FÜR MEDIZIN UND ANDERE VERWENDUNGEN**

SLOWLY CONTRACTING, RIGID CONNECTING ELEMENT FOR MEDICINE AND OTHER USES

ELEMENT DE CONNECTION CONTRACTANT LENTEMENT POUR LA MEDICINE ET D'AUTRES UTILISATIONS

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**HR**

(30) Priorität: **04.05.2005  CH 834052005**

(43) Veröffentlichungstag der Anmeldung:
**01.10.2008  Patentblatt 2008/40**

(73) Patentinhaber: **Synthes GmbH**
**4436 Oberdorf (CH)**

(72) Erfinder:
 • **MAYER, Jörg**
   **CH-5702 Niederlenz (CH)**
 • **GANZ, Jochen**
   **CH-8610 Uster (CH)**
 • **KELLER, Beat**
   **CH-8046 Zürich (CH)**
 • **HERTEL, Ralph**
   **CH-3074 Muri bei Bern (CH)**

(74) Vertreter: **Lusuardi, Werther**
   **Dr. Lusuardi AG**
   **Kreuzbühlstrasse 8**
   **8008 Zürich (CH)**

(56) Entgegenhaltungen:
**EP-A- 0 328 401      EP-A2- 1 199 036**
**WO-A-96/03084      US-A- 4 209 859**
**US-B1- 6 174 333**

 • **GARVIN JOANNE ET AL: "Novel system for engineering bioartificial tendons and application of mechanical load." TISSUE ENGINEERING, Bd. 9, Nr. 5, Oktober 2003 (2003-10), Seiten 967-979, XP002417460 ISSN: 1076-3279**
 • **DATABASE BIOSIS [Online] BIOSCIENCES INFORMATION SERVICE, PHILADELPHIA, PA, US; 1981, HARRIS A K ET AL: "FIBROBLAST TRACTION AS A MECHANISM FOR COLLAGEN MORPHOGENESIS" XP002417469 Database accession no. PREV198172050071 & NATURE (LONDON), Bd. 290, Nr. 5803, 1981, Seiten 249-251, ISSN: 0028-0836**

**Beschreibung**

Technisches Gebiet der Erfindung

Die Erfindung betrifft ein Verbindungselement nach dem Oberbegriff des Anspruchs 1.

Stand der Technik

Bei Bänderrissen oder Sehnenrissen ist es ein weitgehend ungelöstes Problem, beispielsweise eine Sehne an einem Knochen so zu befestigen, dass sich die Verbindung unter Belastung nicht auslockert. Eines der Probleme liegt darin, dass die sich ergebenden Belastungen auf ein Verbindungselement zwischen Knochen und Sehne sehr unterschiedlich sind. Über lange Zeitdauern ist es wünschenswert, dass sich das Verbindungselement kontrahiert, das heisst, dass sich das Verbindungselement zwischen Knochen und Sehne nachspannt. Zusätzlich kann es sich um ein System mit hoher Dämpfung handeln. Durch Bewegungen des Patienten können sich schnell ansteigende hohe Belastungen für das Verbindungselement ergeben, unter welchen die Verbindung nicht funktionell versagen darf, was bedeutet, dass bei kurzfristig auftretende Belastungen der durch das vorgeschlagene Verbindungselement verbundenen Gewebe die Heilung nicht klinisch signifikant beeinträchtigt wird.

Der Stand der Technik erreicht die Verbindung von verschiedenen Strukturen (beispielsweise Sehne mit Knochen) im Körper typischerweise mittels Nahtmaterial, welches steif ist und die auftretenden Kräfte passiv überträgt. Grössere Flächen werden (beispielsweise bei einer Faszienlücke) mittels eines zweidimensionalen Lastträgers, beispielsweise eines Netzes welches verbindbar ist, überbrückt. Unter verbindbar werden eine Reihe von Verfahren verstanden, beispielsweise aber nicht abschliessend vernähbar, heftbar oder klebbar.

Aus der EP 1 284 756 ist beispielsweise der Einsatz von Formgedächtnispolymeren bekannt, um bei einem Gewebeaufbau Muskel, Knorpel oder Nerven zu strukturieren. Aus der US 6 174 333 ist ein Implantat bekannt mit welchem Sehnendefekte repariert werden können. Es enthält eine kontrahierte Gel-Matrix. Aus Garvin et al., Tissue Engineering, Bd. 9, Nr. 5, Seiten 967-979, 2003 ist ein System bekannt, mit dem künstliche Sehnen herstellbar sind. Affen-Flexor-Sehnenzellen werden in einer Collagenmatrix einer uniaxialen Belastung während der Kultivierung unterzogen, was zu einer sehnen-ähnlichen Zellanordnung und hohen Zugfestigkeiten führt. Die Zellen kontrahieren die Matrix zunehmend während der Kultur. Um komplexere Defekte zu überbrücken sind auch flächige oder dreidimensionale Gebilde (beispielsweise ein Beutel um ein Organ) interessant. Ungelöst ist dabei, wie das Auslockern oder Ausreissen einer Verbindung im Gewebe vermieden werden kann. Hier will die Erfindung Abhilfe schaffen.

Ferner ist es bei der Warensicherung, insbesondere im Freien, von Nachteil, dass sich textilbasierte Seile bei Einfluss von Feuchte, wie Tau und Regen, lockern und so Gebinde nicht mehr richtig zusammenhalten. Ausgehend von diesem Stand der Technik liegt die Aufgabe der Erfindung darin, ein Verbindungselement der eingangs genannten Art anzugeben, welches sich über lange Zeitdauern kontrahiert, welches aber andererseits bei kurzzeitigen schnell ansteigenden Belastungen steif ist.

Diese Aufgabe wird erfindungsgemäss mit einem verbindungselement nach Anspruch 1 gelöst.

Eine zeitlich kurze oder zeitlich kürzere Zugbelastung wird als eine solche angesehen, die sich über weniger als 1 Minute auf- und/oder abbaut, insbesondere die kürzer als 10 Sekunden ist. Bei Anwendungen der Erfindung als Reparaturmaterial für den Bewegungsapparat eines Menschen bedeutet dies beispielsweise die Belastung beim Gehen auf das Verbindungsmaterial, welches die Muskeln mit dem Knochen verbindet.

Unter Kontraktion wird auch eine Relaxation des Materials verstanden, beispielsweise im Sinne einer Formänderung des ersten Materials oder einer Zersetzung. Eine solche Formänderung kann auch als eine Deformation angesehen werden, die aber ohne Einwirken einer äusseren Kraft auftritt. Ferner kann das zweite Material aufquellen und durch das erste Material quer zu seiner Längsrichtung gestaucht werden, so dass sich eine Kontraktion ergibt. Insbesondere kann durch das Aufquellen eines Kernmaterials im ersten Material eine Formänderung, beispielsweise durch Änderung des Kreuzungswinkels im Geflecht, erzwungen, welche die Verkürzung des Verbindungselementes zur Folge hat.

Bei Gebinden und Warenbehältern können Seile mit den Merkmalen der Erfindung sicherstellen, dass trotz Witterungseinflüssen die Verpackung sicher durch die sie umgebenden Seile gehalten wird.

Das erfindungsgemässe Verbindungselement eignet sich zum Einsetzen für ein Verfahren zur Heilungsstimulation sowie der Stimulation von biologischen Umbau- und Regenerationsvorgängen von Weichteilen zueinander, wie Sehnen, Bänder, Faszien, Organhöhlen, allgemeines Bindegewebe, Gefässe, Herzklappen, Knorpelgewebe usw., oder von Weichteilen gegenüber Knochen durch die sanfte, aktive, teilweise dynamische Kompression, welche durch Verwendung des hier beschriebenen Materials erreicht werden kann.

Kurze Beschreibung der Zeichnung

Die Erfindung wird nun unter Bezugnahme auf die Zeichnungen näher beschrieben. Es zeigen:

Fig. 1    eine schematische Ansicht eines Teiles eines nicht erfindungsgemässen Verbin- dungselementes kurz nach einem Testeinsatz in-vitro oder in-vivo, das heisst nach einer Implantation, nach

Fig. 2    eine schematische Ansicht des Teiles eines nicht erfindungsgemässen Verbindungs- elementes nach einer langen Zeitdauer nach Beginn des besagten Einsatzes nach Fig. 1,

Fig. 3    eine schematische Ansicht eines Teiles eines nicht erfindungsgemässen Verbin- dungselementes kurz nach einem Testeinsatz in-vitro oder in-vivo, das heisst nach einer Implantation,

Fig. 4    eine schematische Ansicht des Teiles eines nicht erfindungsgemässen Verbindungs- elementes nach einer langen Zeitdauer nach Beginn des besagten Einsatzes nach Fig. 3,

Fig. 5    eine schematische Ansicht eines erfindungsgemässen Verbindungselementes kurz nach einem Testeinsatz in-vitro oder in-vivo, das heisst nach einer Implantation,

Fig. 6    eine schematische Ansicht des erfindungsgemässen Verbindungselementes nach einer langen Zeitdauer nach Beginn des besagten Einsat- zes nach Fig. 5,

Fig. 7    eine schematische Ansicht eines Verbindungselementes kurz nach einem Testeinsatz in-vitro oder in-vivo, das heisst nach einer Implantation, nach einem Aus- führungsbeispiel der Erfindung,

Fig. 8    eine schematische Ansicht des Verbindungselementes nach einer langen Zeitdauer nach Beginn des besagten Einsat- zes nach Fig. 7,

Fig. 9    eine schematische Ansicht eines erfindungsgemässen Verbindungselementes kurz nach einem Testeinsatz in-vitro oder in-vivo, das heisst nach einer Implantation,

Fig. 10   eine schematische Ansicht des Verbindungselementes nach einer langen Zeitdauer nach Beginn des besagten Einsat- zes nach Fig. 9,

Fig. 11   ein schematisches Diagramm für einen beispielhaften Einsatzbereich eines Verbindungselementes nach der Er- findung,

Fig. 12   ein schematisches Diagramm der Fadenspannung gegen die Zeit für ein Verbindungselement in Gestalt eines Fadens nach der Erfindung bei einem Testeinsatz in-vitro oder in-vivo im Vergleich zu einem herkömmlichen Faden,

Fig. 13   eine schematische Ansicht eines Teiles eines Verbin- dungselementes mit einem Faden mit festem Kern nach ei- nem Ausführungsbeispiel der Erfindung,

Fig. 14   eine schematische Ansicht eines Teiles eines Verbin- dungselementes mit einem Faden mit Schlauch-Kern nach einem Ausführungsbeispiel der Erfindung,

Fig. 15   eine schematische Ansicht eines Querschnittes durch ein Verbindungselement mit einem mehrkernigen Faden mit ei- ner äusseren Membran nach einem Ausführungsbeispiel der Erfindung,

Fig. 16   eine schematische Ansicht eines Querschnittes durch ein Verbindungselement mit einem mehrkernigen Faden mit je- weils einer eigenen Membran nach einem Ausführungsbei- spiel der Erfindung,

Fig. 17   eine schematische Ansicht eines Querschnittes durch ein Verbindungselement mit einem mehrkernigen Faden mit ei- ner inneren gemeinsamen Kernmembran nach einem Ausfüh- rungsbeispiel der Erfindung,

Fig. 18   eine experimentell gemessene Kurve, bei der die durch den Faden abgehobene Kraft gegen die Zeit aufgetragen ist,

Fig. 19   zwei experimentell gemessene Kurven, bei der die durch den kontrahierenden Faden eintretende Verkürzung gegen die Körnung für unterschiedliche Sili- kon/Salzverhältnisse aufgetragen ist,

Fig. 20   Überblick über die experimentell bestimmten Verhältnis- se zwischen initialer Verkürzung (in Prozent pro Tag), gegenüber dem Gewichtsverhältnis Silikon zu Salz zur Körnung (in Mikrometer),

Fig. 21   zwei experimentell bestimmte Kurven, bei der die durch den kontrahierenden Faden eintretende Verkürzung in Prozent gegen die Zeit für unterschiedliche Sili- kon/NaCl-Verhältnisse aufgetragen ist, und

Fig. 22   zwei experimentell bestimmte Kurven, bei der die durch den kontrahierenden Faden eintretende Verkürzung in Prozent gegen die Zeit für unterschiedliche TPE/NaCl- Verhältnisse aufgetragen ist.

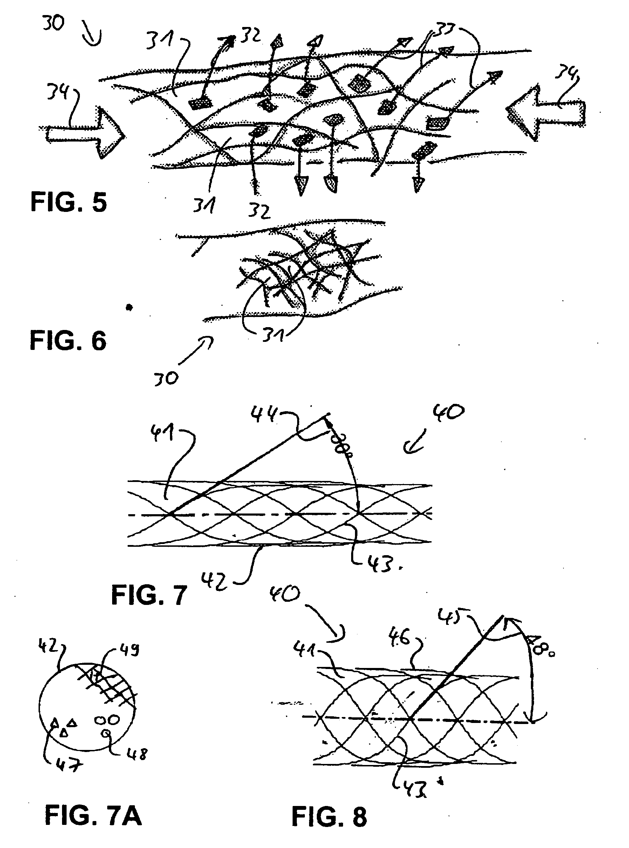

Die Fig. 7 zeigt ein Ausführungsbeispiel eines Verbindungselementes 40. Das Verbindungselement 40 umfasst einen Kern 41, der von einem Mantel 42 umgeben ist. Der Kern 41 ist ein quellfähiges Material, beispielsweise wie unten erwähnt. Der Mantel 42 besteht insbesondere aus einem Netz, beispielsweise aus sich schraubenförmig um den Kern 41 gelegten, insbesondere geflochtenen oder kreuzweise wickelnden Fäden 43, insbesondere aus der Gruppe der für Nahtmaterialien beschriebenen und verarbeiteten bekannten degradablen und nicht degradablen Polymere, welche typischerweise in chirurgischen Nahtmaterialien verwendet werden, wie beispielsweise verstreckte Polyester, Polyamide, Polyolefine, Polyaramide, expandierte oder dichte halogenierte Polymere oder hochfeste Leiterpolymere wie Polyetheretherketon, Kaptone. Die Fig. 7 zeigt das Verbindungselement 40 im Ruhezustand, wobei die Fäden 43 beispielsweise in einem Winkel 44 von 30 Grad zur Längsrichtung des Verbindungselementes 40 ausgerichtet sind. Der Winkel kann in seinem Ausgangszustand beispielsweise zwischen 5 und 50 Grad, insbesondere 10 bis 40 und bevorzugt auf 20 bis 35 Grad ausgelegt sein. Somit fällt das dargestellte Ausführungsbeispiel mitten in dieses Intervall.

Auf schnelle Kraftveränderungen reagiert solch ein Verbindungselement 40 nicht. Dagegen führt eine Quellung des Kerns 42 durch chemo-physikalische Vorgänge zu einer Verdickung des von dem Faden 43 umgebenden Kerns 42. Dadurch verändert sich der Winkel 44 zur Längsrichtung des Verbindungselementes 40 auf einen neuen Winkel 45 von beispielsweise 48 Grad. Dem Netz 46 wird dadurch ein grösserer Durchmesser aufgezwungen und verkürzt sich und damit das gesamte Verbindungselement dadurch. Bei dem Netz handelt es sich bei geflochtenen Fäden um ein Geflecht. Dieser Begriff kann entsprechend überall in der Anmeldung an die Stelle des Wortes Netz treten.

Der Quellvorgang kann zum Beispiel durch einen osmotischen Kern 42 erreicht werden, das heisst einen Kern 42, der mit einer osmotisch aktiven Substanz (beispielsweise Salz, partikuläre Form einer wasserlöslichen Substanz (beispielsweise Sacharide) oder hochkonzentrierte Lösung dieser Substanzen in einem elastischen Schlauch), der entsprechend Wasser aufnimmt.

Beispielsweise kann es sich, wie in Figur 7A in sehr schematischer Weise dargestellt, bei dem Kern 42 um ein fadenförmiges polymeres Material (nicht degradabel oder auch ganz oder teilweise degradabel) handeln, beispielsweise ein thermoplastisches Elastomer (Polyurethan, Polyester), ein vernetztes Elastomer (Silikon, Polyurethan, Elastin, Kollagen) oder ein Gel (Polyethylenglykol, Alginat, Chitosan), in dem Salzkristalle 47 eingelagert sind, wobei die partikuläre Substanz eine Konzentration je nach Korngrösse, Korngrössenverteilung und Agglomartionszustand im Polymer vorteilhafterweise zwischen 5 Prozent und 75 Volumenprozent aufweisen können. Bei der Verwendung von nanoskopischen Partikeln können aufgrund der hohen Partikelzahl aber auch bereits Konzentrationen von weniger als 1% effektiv sein. Der polymere Faden kann aus der Schmelze oder aus der Lösung extrudiert sein und die Partikel sind coextrudiert oder vor der Extrusion der Polymermasse beigemischt. Sie können auch eine Konzentration von 25 bis 60 Prozent aufweisen. Da sich beim Aufnehmen der Umgebungsflüssigkeit um die Partikel einzelne Alveolen bilden, ist die Festigkeit des Kerns (die Fadenfestigkeit wird durch die Eigenschaften der umhüllenden Filamente bestimmt) den Mantel direkt von der Konzentration der Partikel abhängig.

Bei einem anderen Ausführungsbeispiel kann ein Schlauch mit einer beispielsweise PU-Membran von 10 bis 200 Mikrometer vorgesehen sein, in die das expandierende Material oder die osmotisch aktive Substanz beziehungsweise deren hochkonzentrierte Lösung direkt eingefüllt ist. Damit ist bis auf die Packungsdichte 100 Prozent des Volumens mit der osmotisch aktiven Substanz oder dem Salz gefüllt. Bei dem Schlauch kann es sich um PUR, Siloxan, PEG oder andere durchlässige, insbesondere semipermeable Produkte in Gestalt von osmotischen, elastischen oder plastischen sowie geometrisch (Bsp. Strecken axial verlaufender Falten, Plissierungen oder Wellungen) dehnbare Membranen handeln. Insbesondere kann der Schlauch in regelmässigen Abständen eingeschnürt sein, um segmentierte Kammern zu bilden. Dadurch wird erreicht, das der Gesamtfaden beliebig schneidbar bleibt, ohne den beschreibenden Effekt wesentlich zu beeinflussen.

Bei den osmotisch aktiven Substanzen kann es sich um biokompatible anorganische Salze und deren wässrige Lösungen handeln, also beispielsweise um Natriumchlorid (NaCl) oder Calcium - Chlorid, -Carbonat, -Tricalciumphosphat oder es können organische, osmotisch wirkende Moleküle verwendet werden wie beispielsweise niedermolekulare Polysaccharide wie Dextran. Zur Verbesserung der Handhabung wie auch zur weiteren Beeinflussung der Osmosekinetik, können die osmotisch aktiven Substanzen auch in ein biokompatibles Gel oder Hydrogel (beispielsweise aus der Gruppe der Alginate, Chitosane oder deren Copolymere, Polyacrylate, Polyethylenglykol, etc) oder wie oben ausgeführt in ein Elastomer eingebettet werden. Ein zu den osmotisch aktiven Substanzen in seiner grundsätzlichen Wirkung vergleichbarer Effekt kann auch durch die alleinige Verwendung von Hydrogelen erzielt werden. Entsprechend den Fick'schen Gesetzen kommt eine besondere Bedeutung der das quellende System umhüllenden Membran zu, diese beeinflusst entscheidend die Osmosekinetik durch ihre Permeations- respektive Diffusionseigenschaften für H2O wie auch durch ihre Dicke. Selbstverständlich kann die Membran mehrschichtig oder auch mit stabilen oder löslichen Diffusionshemmschichten versehen sein. Im Falle der Verwendung von Hydrogelen kann eine solche membranartige Eigenschaft auch durch eine nach aussen hin stark zunehmenden Vernetzungsdichte erzielt werden. Die Osmose bewirkenden Konzentrationsunterschiede sind zwischen Fadenkern und umgebenden Blut oder inter- und/oder intrastitieller Flüssigkeit des Patienten zu erreichen.

Das Geflecht der Fäden 43 kann durch Textilfäden realisiert werden, wie sie typischerweise für degradable oder nichtdegradable, mono oder polyfilamentäre Nahtmaterialien verwendet werden, beispielsweise verstreckte oder texturierte Polyester, Polyamide, Polyolefine, Polydioxanone. Das Nahtmaterial kann aus einem Quellkern umgeben von den Geflechtfäden wie auch aus mehreren miteinander verflochtenen und jeweils wiederum von einem Fadengeflecht umhüllten quellfähigen Fäden aufgebaut werden. Die Filamentdurchmesser richten sich entsprechen dem Stand der Technik hierbei nach der Feinheit des zu umhüllenden Kernes sowie der Wahl eines mono- oder multifilamentären Hüllgarnes (0.2-200 Mikrometer). Dieser Scherengitter artige wirkende Verkürzungsmechanismus kann auch analog zum einem mit einem Quellkern ausgerüsteten Faden, mit einer quellenden Beschichtung der Strukturfilamente, insbesondere der das Geflecht bildenden oder zusätzlich axial verlaufenden Strukturfilamente erreicht werden. Wie bereits zu den anderen Ausführungsbeispielen angeführt, können durch die Verwendung der genannten Fadenmaterialien auch kontrahierende zwei- oder dreidimensionale textile Gebilde geschaffen werden.

Mit anderen Worten werden dem Verbindungselement 40 langfristig Freiheitsgrade gegeben, so dass ohne Kraftbeanspruchung das Material langsam relaxiert oder kontrahiert. Bei einer Peakbelastung dagegen reagiert das Verbindungselement 40 steif. Selbstverständlich können entsprechend dem Stand der Technik alle mit dem biologischen Gewebe in Kontakt kommende Materialien oder Materialoberflächen chemisch, biochemisch oder biologisch funktionalisiert werden, beispielsweise durch Adsorbieren, Graften oder Freisetzen von biologisch aktiven Substanzen wie Wachstumsfaktoren, Entzündungshemmern, Zytokinen, Rezeptoren oder Rezeptorsequenzen, Antibiotika oder antibiotisch, zytostatisch rsp. bacteriozid, bäcteriostatisch wirkende Substanzen.

Figur 7A zeigt eine erfindungsgemässe weitere Funktionalisierung des Verbindungselementes. In den quellenden Kern 41 werden wirkstoffbeladene Bläschen 48 oder interstitiell gelöste Wirkstoffe 49 eingebracht, die also zwischen Polymerketten eingelagert sind. Durch das Quellen steigt der Druck auf die Bläschen resp. auf die gelösten Wirkstoffe. Damit kann aktiv Wirkstoff aus dem Kern ausgetrieben werden. Durch eine Variation der radialen Verteilungsdichte der Bläschen kann eine zeitlich kontrolliertes Releaseprofil, welches natürlich auch durch den sich aufbauenden Quelldruck beeinflusst wird, angesteuert werden. Falls der Kern mit einer wie oben beschrieben mit einer diffusionskontrollierenden Membran ausgestattet ist, können durch die von der Konzentration respektive von der chemischen Aktivität der abhängigen Transporteigenschaften der Membran, der Stofffluss aus dem Kern heraus zusätzlich beeinflusst werden. Die Bezugszeichen 47, 48 und 49 sind der Übersicht halber an diskreten Orten des Kerns 42 eingezeichnet worden. Prinzipiell können die Salzpartikel 47 isotropisch in dem Kern verteilt sein. Die Wirkstoffe können vorteilhafterweise entweder in Bläschen 48 oder interstitiell gelöst 49 vorgesehen sein, in beiden Ausführungen besteht aber im Gegensatz zur vereinfachten Darstellung eine isotrope Verteilung über den Kern.

Mit anderen Worten, es wird eine Quellwirkung durch Hydratisierung einer Makromolekülstruktur erreicht werden. Eine schlauchförmige, elastische Membrane ist in einer Netzhülle aus steifen Fäden, die den Schlauch schraubenförmig umwickeln, gesteckt. Über diese Netzhülle werden Zugkräfte übertragen. Im Innern des Schlauches befindet sich eine gesättigte Salzlösung. Netzhülle und Membrane werden in eine isotoniosche Lösung gelegt. Durch einen chemophysikalischen Vorgang stellt sich ein Konzentrationsausgleich bis zum Gleichgewichtszustand ein. Durch die Aufnahme des Lösungsmittels wird im Innern der elastischen Schlauchhülle ein grosser Druck aufgebaut welcher eine Quellung des Schlauches zur Folge hat. Es stellt sich ein Kräftegleichgewicht zwischen Innendruck und an der Netzhülle oder Geflechthülle angelegter Zugkraft in Achsrichtung ein. Die als Scherengitter wirkende Netzhülle zieht sich zusammen.

Es ist eine Simulation für eine Abschätzung der Längskontraktionskraft und der Dimensionsänderungen auf Grund des sich einstellenden osmotischen Druckes bei einem gegebenen Konzentrationsunterschied ($\Delta c$) [mol/l] auf den beiden Seiten der Membran für 310 Grad Kelvin durchgeführt worden:

| | |
|---|---|
| Fadendurchmesser am Anfang $d_0$ | $7 \cdot 10^{-4}$ m |
| Ausgangswinkel $\alpha$ | 60 ° |
| Fadenwinkel zur Zugrichtung $\beta$ | 90-$\alpha$ ° |
| Konzentration Körper $C_{Blut}$ | 0.296 mol/l |
| Sättigungskonzentration (NaCl) $C_{sättigung}$ | 6.15 mol/l |

Der osmotische Druck $\Pi$ [Pa] kann für ideal verdünnte Lösungen in einer Vereinfachung wie folgt angegeben werden:

$$\Pi = \Delta c \cdot R \cdot T = \left( c_{S\check{S}ttigung} - c_{Blut} \right) \cdot R \cdot T$$

Radialspannung $\sigma_{Radial}$ [N/m] mit Kesselformel:

$$\sigma_{Radial} = \frac{\Pi \cdot d_{Faden}}{2}$$

Radialkraft ($f_{Radial}$) [N/m] aus Spannung ($\sigma_{Radial}$):

$$\sigma_{Radial} = \frac{F_{Radial}}{\ell_{Umfang}} = \frac{\Pi \cdot d_{Faden}}{2} = f_{Radial}$$

Fadendurchmesser ($d_{Faden}$) :

$$d_{Faden} = d_0 \cdot \frac{\cos \alpha}{\cos \alpha_0}$$

Beziehung Radialkraft ($F_{Radial}$) zu Längskraft ($F_{Längs}$):

$$F_{L\check{s}ngs} = F_{Radial} \cdot \tan \alpha = f_{Radial} \cdot \tan \alpha \cdot \ell_{Umfang} = \frac{\Pi \cdot d_{Faden}}{2} \cdot \tan \alpha \cdot d_{Faden} \cdot \pi$$

$$F_{L\check{s}ngs} = \frac{\Pi \cdot d^2_{Faden}}{2} \cdot \tan \alpha \cdot \pi = \frac{\left(c_{S\check{s}ttigung} - c_{Blut}\right) R \cdot T}{2} \cdot \left(d_0 \cdot \frac{\cos \alpha}{\cos \alpha_0}\right)^2 \cdot \tan \alpha \cdot \pi$$

Druckkraft ($F_{Druck}$) [N] :

$$F_{Druck} = \Pi \cdot A = \Pi \cdot \frac{d^2_{Faden}}{4}$$

Relative Länge (1) [%] :

$$\ell = \frac{\sin \alpha}{\sin \alpha_0}$$

Relatives Volumen (V) [%]:

$$V = \ell \cdot d^2_{Relativ}$$

Resultierende Längenkontraktionskraft ($F_{Res}$) [N]:

$$F_{Res} = F_{L\check{s}ngs} - F_{Druck}$$

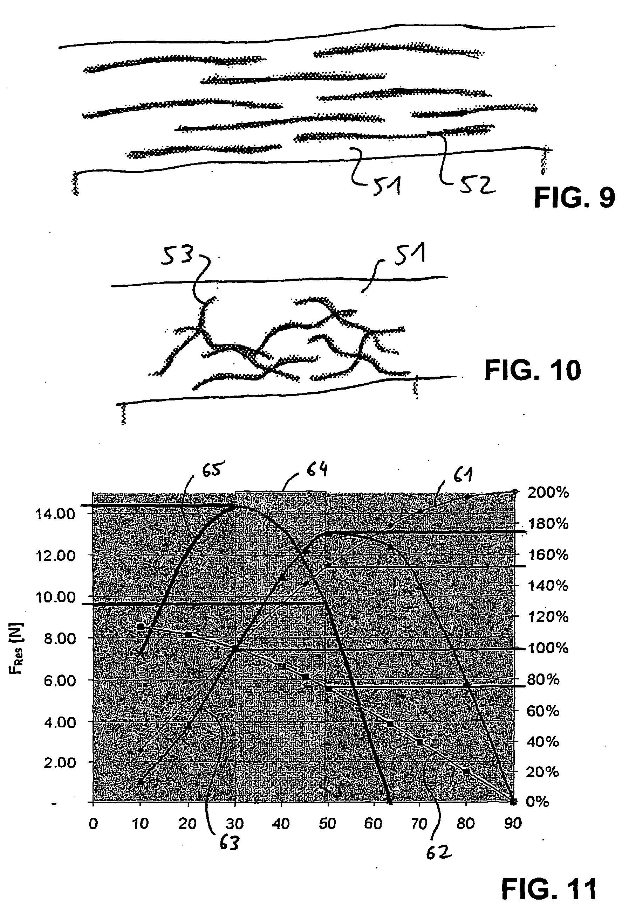

Bei einem Unterschied von $\Delta c$ = 5,8 mol/l hat sich gezeigt, dass die resultierende Längenkontraktionskraft bei einem bestimmten Fadenwinkel von ungefähr 30° und den Ausgangsdimensionen maximal wird. Mit zunehmendem Volumen wird die Fläche und somit die Druckkraft ($F_{Druck}$) grösser, wodurch die resultierende Längenkontraktionskraft abnimmt. Der Anteil der Radialkraft wird ab einem Winkel von 45° grösser als die Längskomponente. Das angestrebte Minimum der Längskraft wird im Beispiel bei einem Fadenwinkel von 48° erreicht. Bei diesem Punkt hat sich der Faden um etwas mehr als 20% verkürzt. Eine entsprechende Darstellung findet sich in Fig. 11.
Die Fig. 11 schliesslich zeigt in schematischer Weise ein Diagramm für ein Ausführungsbeispiel nach der Erfindung, insbesondere einen beispielhaften Einsatzbereich für ein Verbindungselement nach der Erfindung. Auf der X-Achse ist der Fadenwinkel zur Zugrichtung in Grad aufgetragen, der in oben genannten Formeln mit dem Zeichen β versehen worden ist. Auf der Y-Achse ist auf der linken Seite die Kraft Fres in Newton aufgetragen, auf der rechten Seite die relativen Durchmesser (Bezugszeichen 61), Längen (Bezugszeichen 62) und Volumen (Bezugszeichen 63) in Prozent. Der durch den Kasten 64 vorgegebene Fadenwinkelbereich deckt eine Volumenänderung von über 50 Prozent ab. Die entsprechende Kraftkurve 65 zeigt eine nicht allzu unsymmetrische Kraftverteilung, und somit ein nicht starkes Abfallen, um das Maximum der Belastung des Fadenelementes.

Ein nicht erfindungsgemäßes Verfahren zur Behandlung von Gewebe und prosthetischem Material weist den Verfahrensschritt des Verbindens von Gewebe und/oder prosthetischem Material mit einem erfindungsgemässen Verbindungselement auf. Prosthetisches Material kann Faden- oder Netz-Material ohne Nadel umfassen, das als Faden-Schlinge, an einem oder mehreren Nahtankern oder ähnlichen Implantaten vorkonfektioniert befestigt ist. Das prosthetische Material kann auch Fadenmaterial mit einer Nadel umfassen, das vorkonfektioniert als Nadel mit Faden-Schlinge, an einem oder mehreren Nahtankern oder ähnlichen Implantaten befestigt ist. Insbesondere kann ein aus dem Fadenmaterial hergestelltes Band auch über einen Hefter oder Stift oder Nagel beispielsweise mit dem Knochen oder dem Weichgewebe direkt verbunden werden.

Eine Verwendung des sich über die Zeit verkürzenden Verbindungselementes liegt in der Fixation von Sehnen oder Bändern an Knochen. Eine weitere Verwendung des sich über die Zeit verkürzenden Verbindungselementes in Kombination mit Naht-Ankern, fix oder gleitend befestigt, liegt als Schlinge oder als Verbindung von Anker-Haltplättchen ("Parachute") oder Verbindung von mehreren Ankern vor. Die Verwendung des sich über die Zeit verkürzenden Verbindungselementes ist auch bei Säugetieren und anderen Tieren, insbesondere beim Menschen, möglich.

Insbesondere kann die Verwendung des sich über die Zeit verkürzenden Verbindungselementes zur chirurgischen Verwendung im Zusammenhang mit folgenden Applikationen erfolgen: Sehnenrekonstruktion, insbesondere Achillessehnenrekonstruktion oder Rotatorenmanschettenrekonstruktion, Schulterstabilisierungsoperationen am Glenoid, Sehnentransfers, zur Verbindung von Sehnen, Faszien, Bändern oder anderen Weichteilen, Gelenkstabilisierungsoperationen beispielsweise an der Gelenkkapsel, Gelenkstabilisierungsoperationen, insbesondere Acromioclavicular oder Sternoclavikular-Gelenksstabilisierung, Seitenbandrekonstruktionen beispielsweise an Knie, Ellbogen oder Sprunggelenk, Kreuzbandrekonstruktion, Faszienlückenverschluss, Hernienoperationen, Wundverschluss bei offener Wundbehandlung, beispielsweise nach Faszienspaltung, Hautnähten, Rekonstruktion von Sehnen, Knochen oder Weichteilen an Implantaten aller Art, resorbierbar oder nicht-resorbierbar beispielsweise an Prothesen oder Nahtankern, Ligaturen, Fixation/Suspension von Uterus oder Blase, Naht von Darm, Magen, Blase, Gefässen, Trachea, Bronchien oder Oesophagus, und Naht von Faszien.

Dabei kann das sich über die Zeit verkürzende Verbindungselement als Gewebe Verwendung finden. Es kann auch als Beutel zur Umfassung von Organen eingesetzt werden, beispielsweise für das Herz. Die Verwendung des Gewebes ist auch möglich für Faszienlücken.

Das Gewebe kann Verwendung finden als Interponat von Sehnen oder Fasziendefekten. Es kann auch zum Verschluss von Hautdefekten, beispielsweise in Kombination mit künstlicher oder gezüchteter Haut oder anderen Hautverschlussmaterialien, eingesetzt werden oder als Manschette um Gefässe, beispielsweise bei einem Aneurysma, um Gallengänge oder die Gallenblase, um Darmanteile, beispielsweise den Magen. Schliesslich kann das Gewebe auch zur äusseren Anwendung vorgesehen sein, beispielsweise als Stützstrümpfe, Verbrennungsanzug zur Narbenkorrektur oder ähnlichem. Faszienlücken. Ferner kann das Gewebe auch als Interponat für mehrere Sehnen gleichzeigtig dienen, wenn diese mit verschiedenen Anteilen verbunden werden, wie beispielsweise an der Rotatorenmanschette.

Dabei kann es insbesondere vorteilhaft sein, das Material in vorkonfektionierter Form vorliegen zu haben, das heisst in Form der zu ersetzenden oder zu augmentierenden Organen oder Teilen davon, beispielsweise als Kreuzbänder, Sehen, Retinacula, Faszien usw.. Ferner kann das Fadenmaterial mit funktionellen Oberflächenstrukturen beispielsweise mit Widerhaken für Weichteilfixation versehen sein. Schliesslich ist die Verbindung des Fadenmaterials mit Knochennahtankern, gleitend im Anker oder nichtgleitend, zum Knoten oder in Knoten-freier Konfiguration ("knotless"). Dabei ist die Herstellung aus nicht, teilweise oder vollständig resorbierbaren Materialien möglich. Zur Unterscheidung verschiedener Eigenschaften können Verbindungselemente in verschiedenen Farben hergestellt und verwendet werden.

Neben der Einzelanwendung kann auch eine Verwendung in Kombination mit rigiden, ein- oder mehrteiligen Implantaten, z.B. mit einer in sich verschieblichen Kompressionsplatte, welche sich unter Kontraktion des Fadens in gewünschter Weise zusammenzieht, vorgesehen sein.

Neben diesen Anwendungen kann das Verbindungselement auch für die Verbindung von technischen Gegenständen Verwendung finden, beispielsweise für die Verbindung von textilen Stücken oder Befestigungselemente allgemein. Die Beschreibung von der Anwendung von Ausführungsbeispielen in der Medizintechnik bedeutet keine Beschränkung auf diese Anwendung.

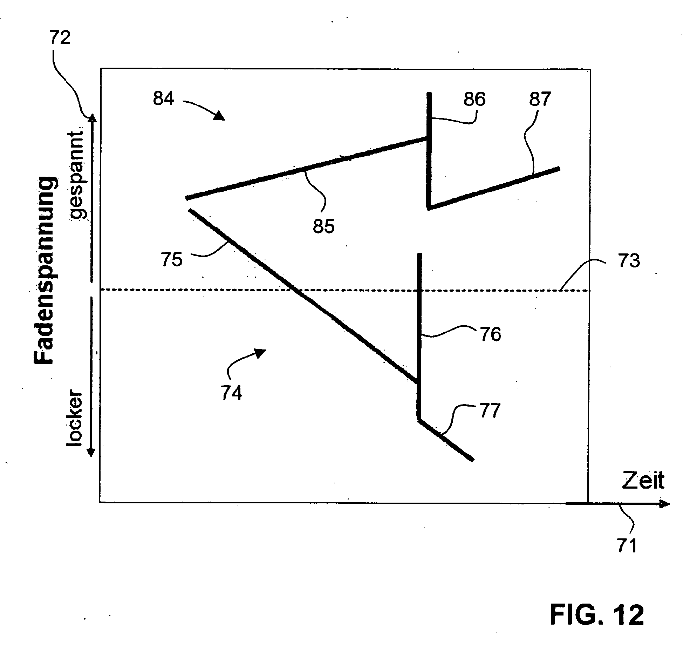

Die Fig. 12 zeigt ein schematisches Diagramm der Fadenspannung 72 gegen die Zeit 71 für ein Verbindungselement in Gestalt eines Fadens 84 nach der Erfindung bei einem Testeinsatz in-vitro oder in-vivo im Vergleich zu einem herkömmlichen Faden 74.

Mit der gestrichelten Linie 73 ist ein willkürliche Schwelle angegeben, oberhalb von der man einen Faden als gespannt (hohe Fadenspannung) und unterhalb von der man einen Faden als eher locker (geringe Fadenspannung) bezeichnen würde.

Die Kurve 74 betrifft einen herkömmlichen Faden, die Kurve 84 einen Faden nach der Erfindung. Nahe am zeitlichen Ausgangspunkt einer Implantierung bei der Befestigung beispielsweise eines Bandes sind die Spannungen beider Fäden vergleichbar. Der herkömmliche Faden verliert allmählich an Spannung, was durch die monoton fallende Gerade 75 dargestellt ist. Bei einem Sturzereignis 76, was auch eine sonstige unsachgemässe Bewegung der Person mit dem angenähten Band sein kann, tritt eine plötzliche grosse Spannung auf, woraufhin zeitlich danach die monoton fallende Gerade 77 nun auf noch niedrigerem Niveau weiter abfällt.

Bei einem erfindungsgemässen Faden 84 dagegen steigt über die Zeit die Fadenspannung monoton 85 an. Dies ist wesentlich, da ein Sturzereignis 86 gleicher Amplitude, hier zeitgleich mit dem Sturzereignis 76, auch zu einer Lockerung des Fadens nach Abebben der kurzzeitig ansteigenden Spannung führt. Das Abfallen ist aber nicht so gross, dass die Spannung nach dem Ereignis wesentlich unter der Ausgangsspannung liegt. Anschliessend findet eine erneute Straffung 87 des Fadens statt, wonach wieder ein höherer Spannungswert erreicht werden kann. Dieser Zyklus kann sich mehrfach wiederholen, um damit Dislokationen der zu verheilenden Gewebeteile bis zum Abschluss des Heilungsprozess, der nach einigen Wochen vollzogen ist, durch kontraktiles wieder Zusammenführen der Gewebeteile kompensieren.

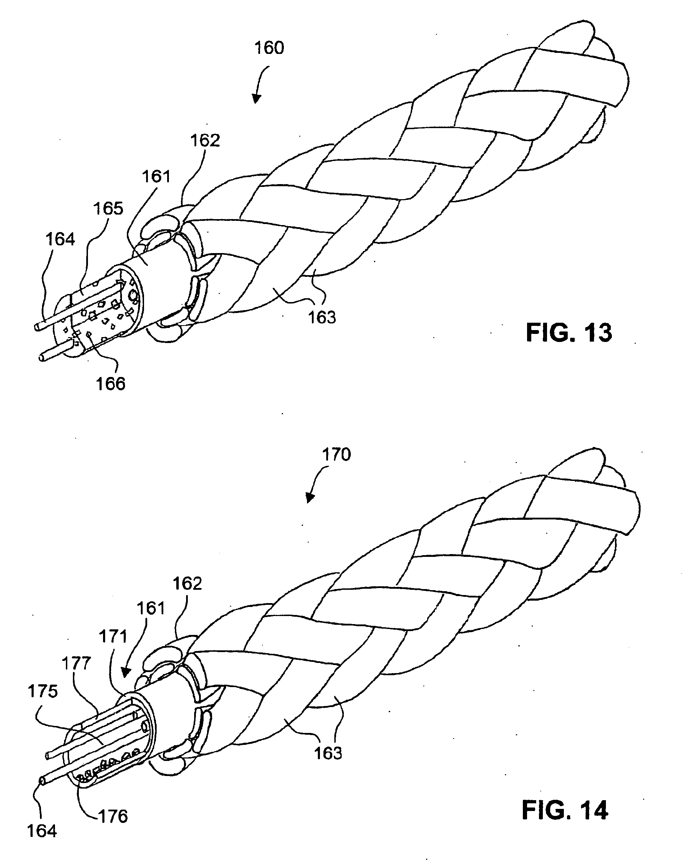

Die Fig. 13 zeigt eine schematische Ansicht eines Teiles eines Verbindungselementes 160 mit einem Faden mit festem Kern nach einem Ausführungsbeispiel der Erfindung. Sie stellt eine spezielle Ausführungsform dar. Der Faden 160 besteht aus einem Kern 161 und einer Netzhülle 162. Die Netzhülle 162 besteht aus hier zwölf verflochtenen Filamenten 163. Bei den Filamenten handelt es sich um Multifilamente, die einen ovalen Raum einnehmen. Dadurch ist es möglich, mit der Verflechtung eine vollständige Abdeckung des Kerns 161 zu erreichen. Anstelle von zwölf können auch mehr (beispielsweise 14, 18 oder mehr) oder weniger (beispielsweise 3, 4, 6 oder 10) Filamente 163 vorgesehen sein. Bei einer höheren Anzahl kann es sich auch um Monofilamente handeln. Der Kern 161 ist hier begrenzt von einer elastisch, plastisch oder geometrich radial dehnbaren Membran und beinhaltet keinen, einen oder mehrere (hier drei) Stichfäden 164 zur Aufnahme von starken Zugbelastungen, wie beispielsweise bei Sturzereignissen 86. In dem Kern 161 ist ferner ein Gel oder eine Matrix 165 vorgesehen, in der osmotisch aktive, partikuläre oder in Bläschen gelöste Substanzen 166, beispielsweise Salzkristalle, eingelagert sein können. Die Salzkristalle können auch durch andere osmotisch aktive Substanzen ersetzt werden. Diese Einlagerungen 166 können dann in der oben genannten Art und Weise durch Osmose Flüssigkeit aufnehmen und durch Ausdehnung des Kerns zu einer Verkürzung, also Straffung des Fadens 160 führen kann. Diese Verkürzung wird durch die kreuzweise Anordnung der HüllenFilamente 163 unterstützt, wohingegen die zentralen Stichfäden die maximale Festigkeit des Fadens 160 festlegen und zugleich die Kompression des Kerns 161 begrenzen.

Die Fig. 14 zeigt eine schematische Ansicht eines Teiles eines Verbindungselementes 170 mit einem Faden mit Schlauch-Kern 161 nach einem Ausführungsbeispiel der Erfindung. Der Faden 170 besteht aus einem Kern 161 und einer Netzhülle 162. Gleiche Bezugszeichen haben in allen Ausführungsbeispielen gleiche oder ähnliche Bedeutung.Der Kern 161 umfasst eine Schlauchmembrane 177, die mit einer Beschichtung 171 versehen sein kann. Die Beschichtung kann wie in den früheren Ausführungen beschrieben, die Diffusionseigenschaften beeinflussen,oder auch die Reibung zwischen Kern und den scherenden Filamenten beispielsweise verringern und damit den Wirkungsgrad des osmotischen Prozesses erhöhen, oder er kann als axial gefaltete, plissierte starre Membran (im Gegensatz zu glatten, elastomeren Membran 177) den Quellprozess begrenzen und ein Ausquellen des Kerns aus dem Geflecht verhindern. Materialbeispiele für Stofftransport: PVD-Beschichtung oder CVD-Beschichtung oder Polymerbeschichtung; für die Expansionsbegrenzung: steifes Strukturpolymer wie Polyamid oder Polyolefin.

Die drei Stichfäden 164 sind von einer gesättigten Salzlösung 175 oder einer anderen osmotisch aktiven Substanz umgeben, bei der weitere partikuläre Salzkristalle 176 zur Aufnahme von weiterer Flüssigkeit zum Erhalt der gesättigten Lösung vorhanden sein können. Die Netzhülle 162 mit den Filamenten 163 ist gleich zum vorherigen Aus-führungsbeispiel ausgestaltet. Die Flüssigkeit kann beispielsweise eine wässrige Lösung, eine hydrophile (beispielsweise höhere Alkohole, DMSO) oder hygroskopische, biokompatible Flüssigkeit oder eine hydrophobe Flüssigkeit (Beispiele Öle) sein. Durch den Grad der Hydrophobie der Flüssigkeit kann die Diffusionsgeschwindigeit und damit die Kinetik des osmotischen Effektes beeinflusst werden. Analog zu den in Fig. 7 beschriebenen Ausführungsform können die Stichfäden auch in eine gelartige oder elastomere Matrix eingebettet sein, in welche zur Erreichung der osmotischen Quellung osmotisch aktive Substanzen in fester oder flüssiger Form partikulär eingelagert sind. Bei ausreichender Eigenstabilität der Matrix, beispielsweise bei einer elastomeren Matrix, kann auch auf die Membran 171 verzichtet werden.

Die Beschichtung könnte auch aus TPU sein. Stichfäden können analog wie oben auch weggelassen werden, in einer anderen Anzahl vorliegen oder aussen am Kern angebracht sein.

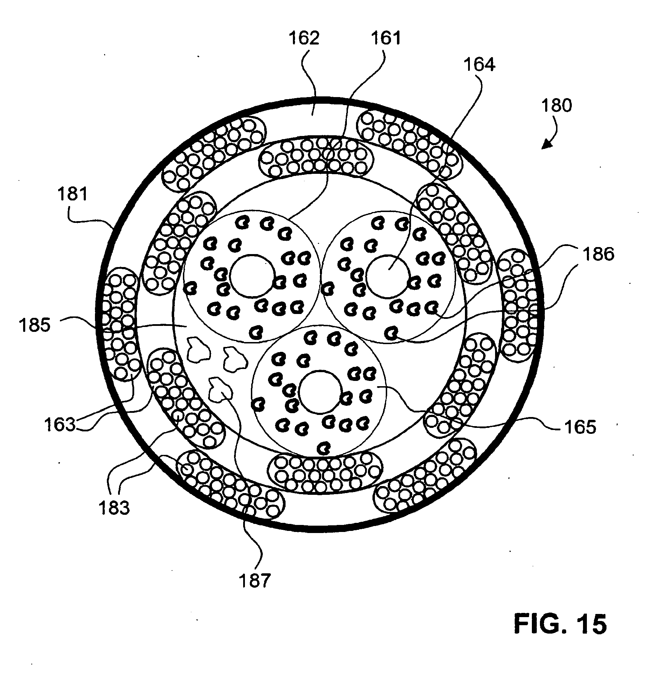

Als Variation der in Fig. 14 gezeigten Ausführungsform, illustrieren Figuren 15-17 verschiedene Ausgestaltungen der Fadenstrukturierung. Fig. 15 zeigt eine schematische Ansicht im Querschnitt eines Verbindungselementes 180 mit einem mehrkernigen 161 Faden mit einer äusseren Membran 181 nach einem Ausführungsbeispiel der Erfindung. Es sind hier drei von einer Osmosemembran umgebenden Kerne 161 vorgesehen, die zum Beispiel jeweils eine Gelfüllung 165 aufweisen, in der Salzkristalle 186 eingebettet sind. Je nach Stärke des zu erzeugenden Fadens können auch vier, fünf, sechs oder mehr Kerne 161 vorgesehen sein, die von einer, beispielsweise aber nicht notwendigweise, Membran umgeben ist, auf der die Filamente 163 der Hülle 162 angeordnet sind. Die (Multi-)Filamente 163 der Hülle 162 umfassen jeweils eine Vielzahl von Einzelfilamenten 183, die sich durch die ausgeübte Zugspannung in der gezeigten Form anlegen. Der Faden 180 insgesamt ist von einer Hülle 181 umgeben, mit der der Faden gegenüber aussen abgedichtet wird. Im Gegensatz zu den Fäden der Fig. 13 oder 14 sind hier die geflochtenen Filamente 163 innerhalb der osmotischen Kammer. Insbesondere kann der Raum 185 auch von der durch Flüssigkeitsaufnahme in ihrer Konzentration abfallenden Lösung gefüllt sein. In dem Raum 185 sowie auch in den durch die Membranen 163 und 181 umschlossenen Räumen können sich auch mit Wirkstoffen gefüllte Bläschen 187 oder direkt Wirkstofflösungen 188 befinden, welche durch die radiale Expansion der Kernstrukturen unter Druck gesetzt werden und damit einen oder mehrere Wirkstoffe austreiben, welcher oder welche über die Membran 181 in das umliegende Gewebe freigesetzt werden. Auch dieses Ausführungsbeispiel kann in spezifischen Merkmalen, wie die anderen Ausführungsbeispiele, durch andere hier beschriebene Merkmale ersetzt sein, beispielsweise, was Anzahl und Lage von Stichfäden angeht.

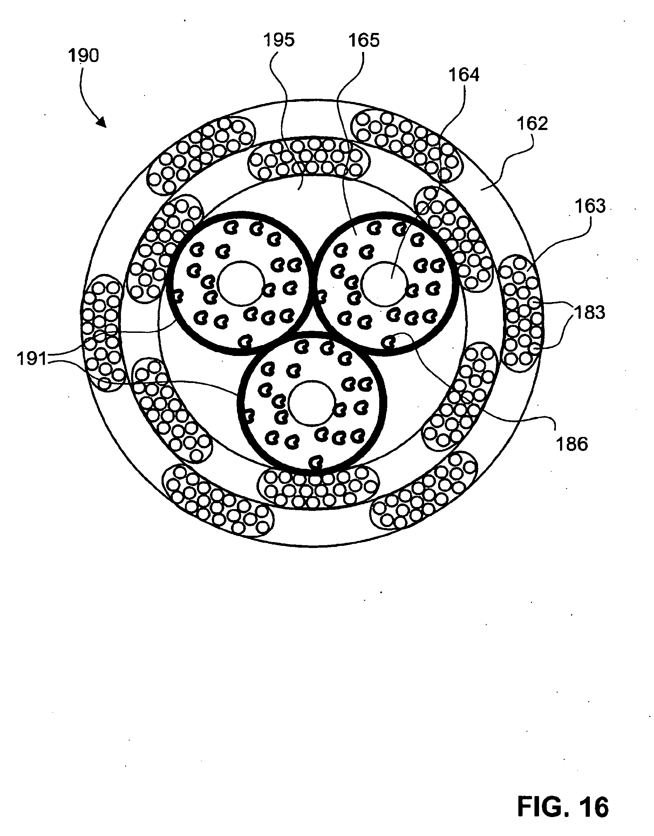

Die Fig. 16 zeigt einen Querschnitt durch ein Verbindungselement 190 mit einem mehrkernigen Faden 190 mit jeweils einer eigenen Kernmembran 191 nach einem Ausführungsbeispiel der Erfindung. Die hier drei Kerne 164 sind jeweils von der osmotischen Flüssigkeit oder Gel respektive elastomeren Polymer 165 mit eingelagerten Salzkristallen 186 umgeben, die insgesamt und je Kern 164 durch eine osmotisch wirkende Membran 191 abgeschlossen ist (oder im Fall des Elastomeren auch nur ein Kern ohne Membrane, anderenfalls ebenso auch möglich ein Kern ohne Stichfaden, wenn diese Struktur - mit oder ohne Membran eigenstabil ist). Um diese abgeschlossenen Kerne 164 herum, die verdrillt sein können oder vorteilhafterweise parallel nebeneinander angeordnet sind, ist die geflochtene Netzhülle 162 vorgesehen. Der Raum 195 zwischen Kernmembranen 191 und Innenfilamenten der Netzhülle 162 kann zuerst bei der Expansion der Membran 191 ausgefüllt werden, bevor es zu der gewünschten Verkürzung des Fadens kommt. Somit ist eine Onset-Zeit in dem Faden vorgesehen, was - im Rückgriff auf die Fig. 12 - zu Beginn zu einem flachen Spannungsanstieg führen kann.

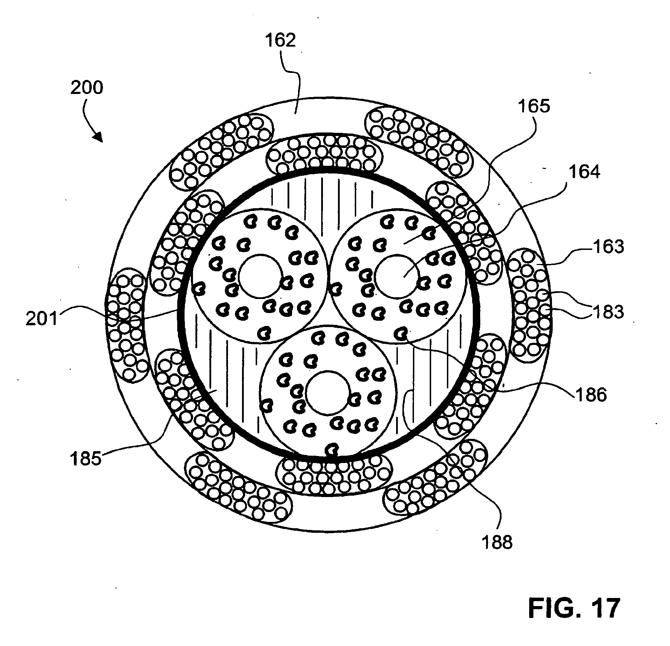

Die Fig. 17 zeigt eine schematische Ansicht eines Querschnittes durch ein Verbindungselement 200 mit einem mehrkernigen Faden 164 mit einer inneren gemeinsamen Kernmembran 201 nach einem Ausführungsbeispiel der Erfindung. Es handelt sich um eine Ausführungsform, die in ihren Merkmalen zwischen denen der Fig. 15 und 16 liegt. Hier ist die Kernmembran 201 innerhalb der geflochtenen Netzhülle 162 angeordnet, aber umgibt nicht jeden einzelnen Kern 164 sondern die Kerne 164 insgesamt, so dass der Raum 185 innerhalb der Membran 201 liegt, dieser kann ebenfalls wie in Fig 15. illustriert, für die Einlagerung von Wirkstoff freisetzenden Systemen genutzt werden. Die Netzhülle 162 umgibt dann diese.

Aus diesen skizzierten Ausführungsbeispielen ist klar, dass die Erfindung nicht auf eine dieser Ausführungsbeispiele beschränkt sein soll. Vielmehr ist auch jede Kombination dieser Merkmale von der Erfindung umfasst. So kann es sich bei den Einzelfäden mit den Stichfäden 164 um eine flüssige, gelartige oder polymere Substanz handeln. Es könnte aber auch kein Stichfaden als solcher sondern nur die Matrix haben; mehrere Matrixfäden im Kern könnten gerade bei relativ grosskalibrigen Fäden von Vorteil sein, weil sie den Faden weicher werden lassen, zudem wird die Diffusionskinetik bei meheren kleinkalibrigen Fäden im Vergleich zu einen grosskalibrigen beschleunigt.

Die Anzahl der Stichfäden (hier drei) ist zwischen keinem und mehreren Dutzend änderbar. Die Netzhülle 162 besteht hier jeweils aus einem Multifilament 163 mit jeweils neunzehn Monofilamenten 183. Es ist klar, dass sowohl die Art der Multifilamente 163 als auch die Anzahl der Monofilamente 183 veränderbar ist. Erstere Anzahl kann insbesondere zwischen drei und zehn gewählt werden, letztere Anzahl zwischen zehn und über einhundert, wobei bei einer relativ starren inneren Membran auf eine vollständige Überdeckung der Netzhülle unter Umständen verzichtet werden, da dann die Membran nicht zwischen den Überdeckungsfehlstellen austreten kann. Wesentlich ist eine Membranhülle, die die Diffusion zulässt, aber gleichzeitig den Druckunterschied begrenzt, so dass ein Versagen der Membran sicher vermieden werden kann. Dazu dienen auch die Stichfäden 164, die plötzliche Zugbelastungen aufnehmen und eine übermässige Kompression des Kerns des Fadens bei Sturzereignissen sicher vermeiden. Damit ist dem Fachmann klar, dass Merkmale von allen beschriebenen Ausführungsbeispielen direkt miteinander kombinierbar und austauschbar sind.

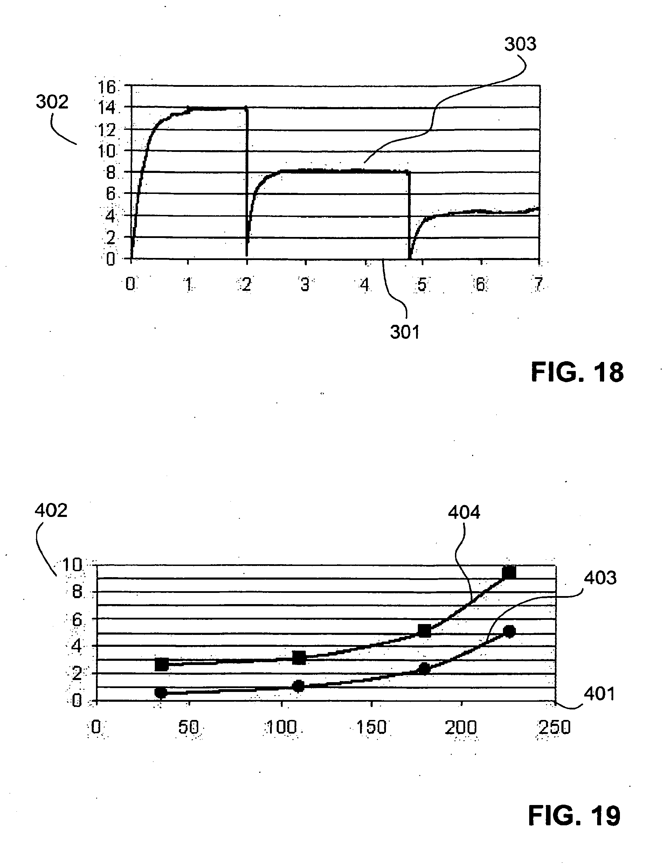

Mit entsprechend präparierten Fäden sind verschiedene Versuche durchgeführt worden, die als Beispiele für mögliche Ausführungsbeispiele aufgeführt werden. So ist die Kurve der Fig. 12 durch einen Test im Abheben eines Gewichts verifiziert worden. Dies zeigt die Fig. 18 in einer Kurve 303, bei der die abgehobene Kraft 302 gegen die Zeit 301 aufgetragen ist. Es handelt sich bei dem untersuchten Faden um einen Faden nach Fig. 13 (jedoch ohne Stichfäden 164 und Membran 161) mit einem Gewichtsverhältnis Silikonmatrix zu Salz von 1:1, wobei die Korngrösse der Kristalle 166 kleiner als 70 Mikrometer ist und wobei der Faden in destilliertem Wasser mit einer Temperatur von 37 Grad Celsius fest eingespannt ist. Die Fadenspannung wird kontinuierlich gemessen. Die Zugkraft (Fadenspannung) verstärkt sich in weniger als einem Tag auf über 12 Newton, um dann in einen durch die Hülle begrenzten Gleichgewichtszustand überzugehen. Der Faden ist nach zwei Tagen bewusst gelockert worden, was beispielsweise einem Sturz des Patienten mit einem durch einen solchen Faden angenähten Band entspricht. Die Lockerung kann einfach durch eine Verlängerung erreicht werden. Dadurch beginnt der Fadenspannungsaufbau erneut, diesmal allerdings in geringerem Ausmass, die aufgebaute Federspannung erreicht nur noch eine Zugkraft von ca. 8 Newton. Hier liess man den Gleichgewichtszustand knapp drei Tage anhalten, um eine erneute Lockerung vorzunehmen. In diesem dritten Bereich können dann nach 5 Tagen und mehr immer noch Spannung für etwas über 4 Newton aufgebaut werden, wobei die nun flache Kurve 303 anzeigt, dass die maximale Spannung in dem Faden aufgebaut worden ist.

Die Fig. 19 zeigt eine schematische Ansicht von zwei Kurven 403 und 404, bei der Zeit, bis die maximale Verkürzung 402 durch den kontrahierenden Faden eingetreten ist, gegen die Körnung 401 für unterschiedliche Silikon/Salzverhältnisse aufgetragen ist. Die Fig. 18 hat ein Silikon/Salzverhältnis von 1:1 aufgewiesen. Das heisst, dass die Füllung 165 zu Kristallen 166 in einem Gewichtsverhältnis von 1:1 gestanden haben. Die zwei Kurven 403 beziehungsweise 404 zeigen die Dauer bis zur maximalen Verkürzung als Funktion der Körnung 401 der Salzkristalle, wobei diese Zeitdauer bei einem Verhältnis von 2 : 1 Silikon zu Salz kürzer ist als bei einem Verhältnis von 0,71 : 1, wenn also mehr Salz im Kern enthalten ist. Es ist zu festzuhalten, dass es sich hier nicht um eine Gesetzmässigkeit sondern um experimentelle Ergebnisse handelt, deren Charakteristik sich in Abhängigkeit von weiteren Parametern, wie beispielsweise der örtlichen Verteilung der Salzkristalle, der Agglomeratbildung sowie der Gefügestruktur des Polymeren noch signifikant ändern kann. Im kleinen Körnungsbereich von unter 50 bis ca. 150 Mikrometer ergeben sich kaum Unterschiede, während bei grösserer Körnung die Zeitdauer bis zur maximalen Verkürzung rasch ansteigt.

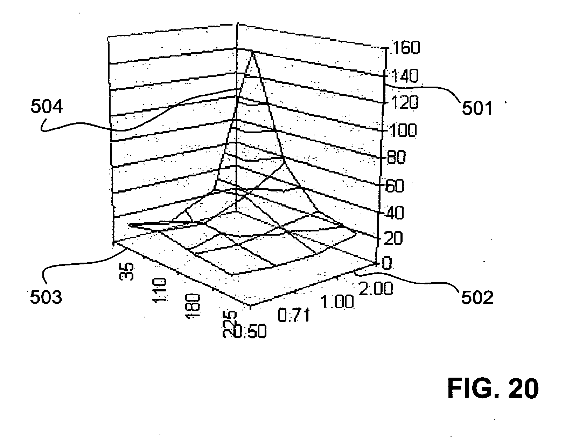

Einen Überblick über die Verhältnisse zwischen initialer Verkürzung 501 (in Prozent pro Tag), gegenüber dem Gewichtsverhältnis Silikon zu Salz 502 zur Körnung 503 (in Mikrometer) wird durch die dreidimensionale Kurve 504 der Fig. 20 dargestellt. Viel Silikon gegenüber dem Salz und kleine Körnungen ergeben einen schnelle Verkürzung in der Zeit, während sowohl grosse Körnungen als auch ein höherer Salzanteil zu einer kleineren Verkürzung pro Tag führen. Der Fachmann kann daher durch entsprechende Wahl der einzelnen Komponenten das Verhalten des Fadens in einem weiten Bereich einstellen.

Insbesondere ist dabei folgendes zu beachten. Verbindungselemente in Gestalt eines Fadens können in Durchmessern bis hinab zu 50 Mikrometer (und darunter) hergestellt werden, wenn es um chirurgische Applikationen geht. Für dickere Fäden kann dann eine Zwillich- oder Drillichstruktur, allgemein ausgedrückt eine mehrfädige Struktur ausgebildet werden. Der Vorteil von diesen liegt darin, dass sich bei den so hergestellten Verbindungselementen eine höhere Festigkeit durch Reibung der einzelnen Fäden ergibt, andererseits aber aus dem gleichen Grund der Vielzahl von verzwirnten, verdrillten oder sonstwie verdrehten Fäden eine reduzierte Steife vorliegt.

Bei einem Faden von 50 Mikrometer Durchmesser sind Pulver der Salzkristalle von unter 100 Nanometer bis 1 Mikrometer sinnvoll.

Aus jedem dieser Kristalle bilden sich kleine Zentren der osmotischen Tätigkeit. Insbesondere sollten diese Zentren, bei denen es sich um um solche Salzkerne gebildete Bläschen handelt, um einen Faktor von ungefähr 10 kleiner sein als der Durchmesser des quellenden Kerns. Mit vielen kleinen Zentren ist eine sicherere osmotische Tätigkeit verbunden, als mit einigen wenigen grossen Kristallen. Die Geschwindigkeit der sich ergebenden Verkürzung solcher Fäden entsprechend der Lehre ihrer Konstruktion wird vorteilhafterweise durch die Eigenschaften des verwendeten Polymermaterials für den quellenden Kern eingestellt.

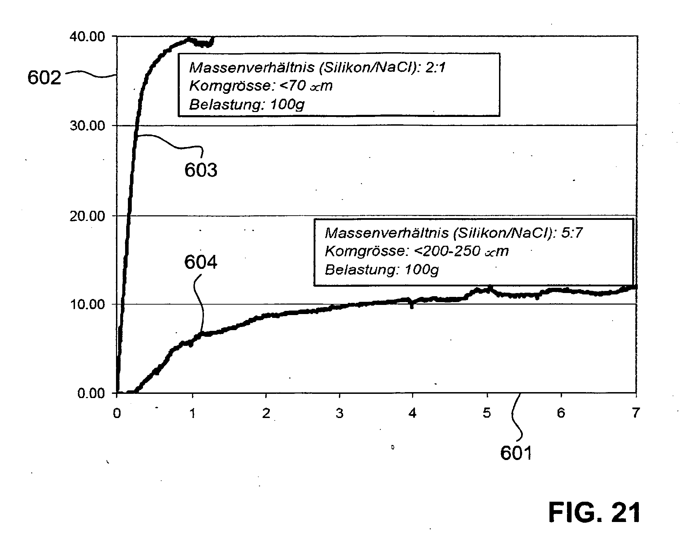

Die Fig. 21 beziehungsweise 22 zeigen eine Ansicht von jeweils zwei gemessenen Kurven, bei der die durch den kontrahierenden Faden eintretende Verkürzung 602 in Prozent gegen die Zeit 601 für unterschiedliche Silikon/NaCl (Salz)-Verhältnisse 603/604 beziehungsweise für unterschiedliche TPE/NaCl(Salz)-Verhältnisse 605/606 aufgetragen ist.

Es ist zu erkennen, dass bei einem Silikonfaden mit einem Massenverhältnis Silikon zu NaCl von 2:1 bei einer durchschnittlichen Korngrösse der Salzkristalle von kleiner 70 Mikrometer und einer konstanten Fadenspannung von 1 Newton, ein Gleichgewichtszustand der Kurve 603 nach ca. einem Tag auf hohem Verkürzungsniveau eintritt. Dagegen ist eine vierfach kleinere Verkürzung bei einem Massenverhältnis Silikon zu NaCl von 5:7 bei einer durchschnittlichen Korngrösse der Salzkristalle von kleiner 200 bis 250 Mikrometer und einer weiterhin gleichen konstanten Fadenspannung von 1 Newton in der Kurve 604 zu erkennen, die nach ca. vier Tagen erreicht wird. Der Kern hatte einen Durchmesser von 0,7 Millimeter.

In einem zeitlich anderen Horizont sind die Versuche mit TPE-Fäden abgelaufen. Es ist zu erkennen, dass bei einem TPE-Faden mit einem Massenverhältnis TPE zu NaCl von 1:1 bei einer durchschnittlichen Korngrösse der Salzkristalle von kleiner 160 bis 200 Mikrometer und einer konstanten Fadenspannung von 1 Newton (das heisst wie beim anderen Versuch), ein Gleichgewichtszustand der Kurve 605 nach ca. zwanzig bis fünfundzwanzig Tagen auf sehr kleinem Verkürzungsniveau von einem Prozent eintritt. Dagegen ist eine achtfach grössere Verkürzung bei einem Massenverhältnis TPE zu NaCl von 2:1 bei einer durchschnittlichen Korngrösse der Salzkristalle von kleiner 70 bis 150 Mikrometer und einer weiterhin gleichen konstanten Fadenspannung von 1 Newton in der Kurve 604 zu erkennen, die auch nach über zwanzig Tagen noch keinen Gleichgewichtszustand erreicht hat.

Somit es ist es für den Fachmann ersichtlich, dass er bei Einsatz von TPE- und Silikon-Fadenkernen mit unterschiedlichem Salzgehalt und Körnung eine geeignete Verkürzung zwischen 40 Prozent in einem Tag und einem Prozent in fünf Tagen einstellen kann, was einem Unterschied in der Geschwindigkeit mit einem Faktor 200 entspricht. Diese Werte können zudem durch entsprechende Verwendung von Membranen (mehr oder weniger durchlässig; mehr oder weniger flexibel in der Ausdehnung) moduliert werden. Die hier dargestellten Ergebnisse mit Fäden mit einer Seele sind auf die anderen Ausführungsbeispiele entsprechend übertragbar.

Neben Silikon, welches in verschiedenen Qualitäten einsetzbar ist, gilt dies in noch grösserem Masse für Fäden mit TPE-Füllung, das heisst für thermoplastische Fäden. Diese thermoplastischen Elastomere lassen sich sehr leicht formen, da sie bei der Verarbeitung den plastischen Zustand durchlaufen. Sie lassen sich insbesondere in Härten von 5 Shore A bis 90 Shore D herstellen. Ihre Fließfähigkeit, sowie ihre Dichte und andere Eigenschaften lassen sich durch Compoundierung mit verschiedensten Füllstoffen und Additiven einstellen. TPE-V weist dafür gute gummiartige Eigenschaften auf, beispielsweise Ethylen/Propylen-Terpolymer/Propylen, vernetzt oder Naturkautschuk/Polypropylen.

Damit umfasst das zweite Material ein quellendes Material, insbesondere ein hygroskopisches Material, wie NaCl, welches den Vorteil hat, im Körper in leichter Weise einen Gleichgewichtszustand aufzubauen, ohne den Körper des Patienten durch die osmotische Tätigket zu stark zu belasten. Das Aufquellen des zweiten Materials wird durch Osmose erreicht, das heisst durch Diffusion von Wasser aus dem das Verbindungselement umgebenden Flüssigkeit enthaltenden Raum (in vitro zum Beispiel Wasser oder physiologische Salzlösung in einem Becherglas; und in vivo durch die den Implantatsort eines Fadens umgebenden Körperflüssigkeiten) durch eine semipermeable beziehungsweise selektiv permeable Membran, die der Fachmann entsprechend auswählt.

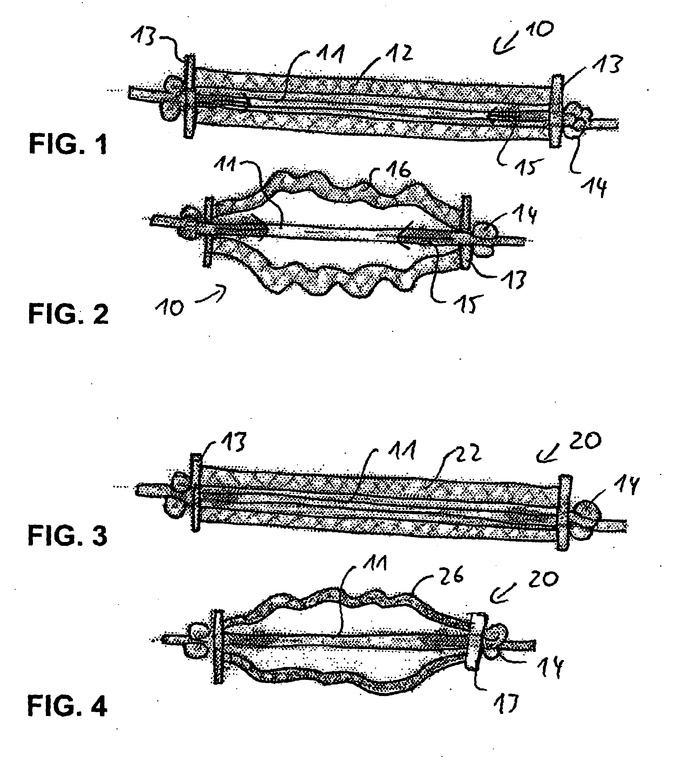

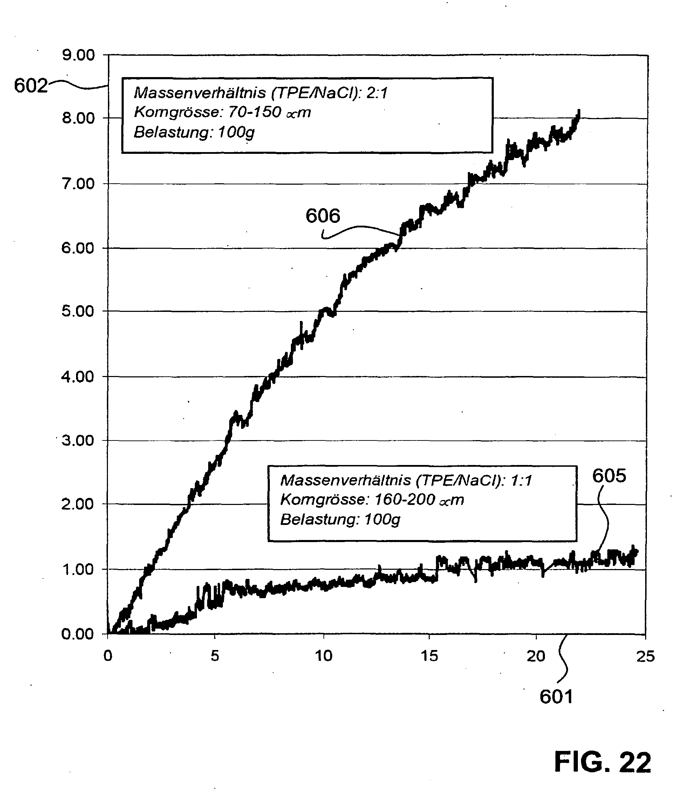

Die Ausführungsbeispiele der Fig. 12 bis 22 stehen in direktem Zusammenhang mit der Offenbarung zu den Fig. 1 bis 11. Dabei kann das erste Material als der oder die Stichfäden angesehen werden und/oder axiale Fäden in der Netzhülle sowie die Membran oder axiale Faden-Verstärkungen in der oder den Membranen selber. Das zweite Material, welches sich über eine längere Zeitdauer langsam kontrahiert, ist dabei die eine oder mehrere Kammern 165, 185, in denen die zur Quellung beitragenden Kristalle eingelagert sind. Eine Scherung des das zweite Material umgebende erste Material ergibt sich insbesondere durch Vorsehen von geflochtenen Netzhülle. Unter der langsamen Kontraktion des zweiten Materials über eine zweite, gegenüber der ersten Zeitdauer längeren Zeitdauer wird auch die Kombination der Wirkung mit dem ersten Material verstanden, so wie sie bei dem geflochtenen Faden entsteht. Wesentlich ist nur, dass sich für jeweils zwei entsprechende örtliche Bezugspunkte in dem Element über einen Zeitraum der Abstand verkürzt, sich also eine Spannung zwischen diesen beiden Punkten aufbaut. Sofern diese Punkte nicht fest eingespannt sind, verkürzt sich der Abstand zwischen diesen, was einer Kontraktion des zweiten Materials entspricht.

Es ist aber auch möglich, die Membran selber in kurzen axialen Abständen von 3 bis 10 facher Länge des Normdurchmessers eines Fadens in Nahtstellen zusammenzuschweissen, um einzelne axial definierte Kammern zu erzeugen, die sich bei Schwellung per se in ihrer Länge verkürzen. Insofern sind sämtliche Verwendungen für das offenbarte prosthetische Material auch für Verbindungselemente umfasst, die nach der Lehre der Fig. 12 bis 22 ausgestaltet sind.

Unter der zeitlich kürzeren Belastung kann auch eine Verkrampfung oder ein Spasmus die Gewebedislokation aufbauen, wobei eine Peakbelastung und ein etwas langsamerer Aufbau möglich ist.

Bezugszeichenliste:

| | |
|---|---|
| 10 | Teil eines Verbindungselement |
| 11 | Seele |
| 12 | Mantel (ursprünglich) |
| 13 | Verbindungskonstruktion (Netz) |
| 14 | Knoten |
| 15 | Pfeil |
| 16 | Mantel (verformt) |
| 20 | Teil eines Verbindungselement |
| 22 | Mantel (ursprünglich) |
| 26 | Mantel (verformt, in Degradation) |
| 30 | Verbindungselement |
| 31 | Molekül |
| 33 | Abgaberichtung |
| 34 | Pfeil |
| 40 | Verbindungselement |
| 11 | Seele |
| 42 | Mantel (ursprünglich) |
| 43 | Netz |
| 44 | Winkel (zu Beginn) |
| 45 | Winkel (später) |
| 46 | Netz |

| 47 | Salzkristalle |
|---|---|
| 48 | wirkstoffbeladene Bläschen |
| 49 | Wirkstoffe |
| 50 | Verbindungselement |
| 51 | Grundmaterial |
| 52 | Fadenmoleküle (zu Beginn) |
| 53 | Fadenmoleküle (später) |
| 61 | resultierende Durchmesserveränderung |
| 62 | resultierende Längenänderung |
| 63 | resultierende Volumenänderung |
| 64 | Fadenwinkelbereich |
| 65 | resultierende Kraftveränderung |
| 71 | Zeit |
| 72 | Fadenspannung |
| 73 | Übergang gespannt⇔locker |
| 74 | herkömmlicher Faden |
| 75 | Entspannung |
| 76 | Sturz |
| 77 | weitere Entspannung |
| 84 | Faden nach der Erfindung |
| 85 | Straffung |
| 86 | Sturz |
| 87 | weitere Straffung |
| 160 | Faden |
| 161 | Kern |
| 162 | Netzhülle |
| 163 | Hüllenfilamente |
| 164 | Stichfäden |
| 165 | Matrix |
| 166 | Salzkristall |
| 170 | Faden |
| 171 | Beschichtung |
| 175 | Salzlösung |
| 176 | Salzkristalle |
| 177 | Schlauchmembrane |
| 180 | Faden |
| 181 | äussere Membrane |
| 183 | Monofilament |
| 185 | Raum mit osmotischer Flüssigkeit |
| 186 | Salzkristalle . |
| 187 | Bläschen mit Wirkstoff |
| 188 | Wirkstofflösungen |
| 190 | Faden |
| 191 | Kernmembran |
| 195 | Raum zwischen Kernen und Hülle |
| 200 | Faden |
| 201 | Mehrkernmembran |
| 301 | Zeit |
| 302 | abgehobene Kraft |
| 303 | Kurve |
| 401 | Körnung |
| 402 | Verkürzung |
| 403 | Kurve bei höherem Salzgehalt in Silikon |
| 404 | Kurve bei niedrigerem Salzgehalt in Silikon |
| 501 | initialer Verkürzung 501 (in Prozent pro Tag), |
| 502 | Gewichtsverhältnis Silikon zu Salz |
| 503 | Körnung (in Mikrometer) |
| 504 | dreidimensionale Kurve |

601 Zeit (in Tagen)
602 Verkürzung (in Prozent)
603 - 606 Kurven für verschiedene Fäden

**Patentansprüche**

**1.** Verbindungselement (40, 160, 170, 180, 190, 200), insbesondere ein Nahtmaterial für die chirurgische Anwendung, bestehend aus einem ersten Material (42, 162, 163, 164, 181, 183), welches bei einer Beaufschlagung auf einander entfernt liegenden Seiten mit einer zeitlich kürzeren Zugbelastung im wesentlichen steif ist, wobei das erste Material eine netzartige Struktur umfasst, und aus einem zweiten, mit dem ersten Material (42, 162, 163, 164, 181, 183) verbundenen Material (41, 165/166, 175/176, 165/186), wobei das Verbindungselement über eine zweite, gegenüber der ersten Zeitdauer längeren Zeitdauer, langsam kontrahiert, **dadurch gekennzeichnet, dass** das Verbindungselement (40, 160, 170, 180, 190, 200) durch eine Quellung des zweiten Materials (41) und eine **dadurch** resultierende Scherung des mit dem zweiten Material (41) in Verbindung stehenden ersten Material (42) kontrahiert, wobei das erste Material (40, 160, 170, 180, 190, 200) das zweite Material (41, 165/166, 175/176, 165/186) umgibt.

**2.** Verbindungselement nach Anspruch 1, **dadurch gekennzeichnet, dass** das zweite Material ein aus der folgender Gruppe ausgewählter Kern ist:

- ein Kern umfassend ein fadenförmiges polymeres Material, wie ein thermoplastisches Elastomer oder ein vernetztes Elastomer oder ein Gel, in dem Salzkristalle (47) eingelagert sind
oder
- ein Kern mit einem Schlauch, in welchem ein expandierende Material oder eine osmotisch aktive Substanz eingefüllt ist; oder
- ein Kern (161), welcher durch eine elastische, plastische oder geometrisch radial dehnbare Membran begrenzt ist, wobei im Kern (161) ferner ein Gel oder eine Matrix (165) vorgesehen ist, in der osmotisch aktive, partikuläre oder in Bläschen gelöste Substanzen (166) eingelagert sind.

**3.** Verbindungselement nach Anspruch 2, **dadurch gekennzeichnet, dass** der Schlauch in regelmässigen Abschnitten eingeschnürt ist, um segmentierte Kammern zu bilden.

**4.** Verbindungselement nach Anspruch 1, **dadurch gekennzeichnet, dass** das zweite Material von einer semipermeablen Membran bzw. einer selektiv permeablen Membran oder von einer Osmosemembran umgeben ist.

**5.** Verbindungselement nach einem der vorstehenden Ansprüche, **dadurch gekennzeichnet, dass** sich die zeitlich kürzere Zugbelastung über eine erste Zeitdauer von weniger als 1 Minute auf- und/oder abbaut.

**6.** Verbindungselement nach einem der vorstehenden Ansprüche, **dadurch gekennzeichnet, dass** die erste Zeitdauer gegenüber der zweiten Zeitdauer um mindestens zwei Grössenordnungen kürzer ist.

**7.** Verbindungselement nach einem der vorstehenden Ansprüche, **dadurch gekennzeichnet, dass** das Verbindungselement sich bei Beaufschlagung unter einer Schwellspannung zeitlich langsam kontrahiert oder nachspannt, bei kurzeitiger Zugbelastung über der Schwellspannung aber im wesentlichen steif ist.

**8.** Verbindungselement nach einem der vorstehenden Ansprüche, **dadurch gekennzeichnet, dass** das Verbindungselement ein Nahtmaterial ist, welches aus mehreren miteinander verflochtenen und jeweils wiederum von einem Fadengeflecht umhüllten quellfähigen Fäden aufgebaut wird.

**9.** Verbindungselement (40, 160, 170, 180, 190, 200) nach Anspruch 1 **dadurch gekennzeichnet, dass** das erste Material aus im wesentlichen axial ausgerichteten Fäden (164) und/oder das zweite Material axial umgebenden Flächenbestandteilen (161) besteht, und dass das zweite Material ein quellendes Material umfasst, insbesondere ein hygroskopisches Material oder ein hydrophobes Material mit einer hygroskopischen Einlagerung.

**10.** Verbindungselement nach einem der Ansprüche 1-9, **dadurch gekennzeichnet, dass** das zweite Material von einer semipermeablen Membran bzw. einer selektiv permeablen Membran oder von einer Osmosemembran umgeben ist.

11. Prosthetisches Material bestehend aus einem textilen Gebilde wie einem Faden, Vliess, Gewebe, Geflecht, Gestrick, Gestick, Netz oder ähnlichem aus einer Vielzahl von Verbindungselementen nach einem der Ansprüche 1 bis 10.

12. Verwendung des Verbindungselementes nach einem der Ansprüche 1-10 als textiles Gebilde, vorzugsweise als Gewebe.

13. Verbindungselement nach einem der Ansprüche 1-10 in Form eines textilen Gebildes als Beutel zur Umfassung von Organen.

14. Verbindungselement nach einem der Ansprüche 1-10 in Form eines textilen Gebildes Faszienlücken.

15. Verbindungselement nach einem der Ansprüche 1-10 in Form eines textilen Gebildes als Interponat von Sehnen oder Fasziendefekten.

16. Verbindungselement nach einem der Ansprüche 1-10 in Form eines textilen Gebildes als Verschluss von Hautdefekten.

17. Verbindungselement nach einem der Ansprüche 1-10 in Form eines textilen Gebildes als Manschette für Gefässe.

18. Verbindungselement nach einem der Ansprüche 1-10 in Form eines textilen Gebildes als Stützstrümpfe, Verbrennungsanzug oder Narbenkorrektur.

19. Nicht medizinische Verbindungselementes nach einem der Ansprüche 1 bis 10 als Spannfaden, zur Herstellung eines Spannnetzes oder einer Textilmembran für die Verpackung oder Warensicherung.

## Claims

1. A joining element (40, 160, 170, 180, 190, 200), especially a suture material for surgical use, consisting of a first material (42, 162, 163, 164, 181, 183) that is substantially rigid when a shorter lasting tensile load acts upon sides remote from each other, wherein the first material comprises a net-like structure, and of a second material (41, 165/166, 175/176, 165/186) connected with the first material (42, 162, 163, 164, 181, 183), wherein the joining element contracts slowly during a second period of time that is longer than the first period of time, **characterized in that** the joining element (40, 160, 170, 180, 190, 200) contracts by a swelling of the second material (41) and a thereby resulting shearing of the first material (42) connected with the second material (41), wherein the first material (40, 160, 170, 180, 190, 200) surrounds the second material (41, 165/166, 175/176, 165/186).

2. The joining element according to claim 1, **characterized in that** the second material is a core selected from the following group:

   - a core comprising a filamentary polymer material, like a thermoplastic elastomer or a crosslinked elastomer or a gel, in which salt crystals (47) are incorporated; or
   - a core with a tube, into which an expanding material or an osmotically active substance is filled; or
   - a core (161) which is delimited by an elastically, plastically or geometrically radially extensible membrane, wherein the core (161) is further provided with a gel or a matrix (165), in which osmotically active, particulate substances (166), or substances (166) dissolved in vesicles are incorporated.

3. The joining element according to claim 2, **characterized in that** the tube is narrowed at regular intervals to form segmented chambers.

4. The joining element according to claim 2, **characterized in that** the second material is surrounded with a semipermeable membrane, respectively with a selectively permeable membrane or with a osmosis membrane.

5. The joining element according to one of the claims 1 to 4, **characterized in that** the shorter-lasting tensile load builds up and/or abates over a first period of time of less than 1 minute.

6. The joining element according to one of the claims 1 to 5, **characterized in that** the first period of time is shorter than the second period of time by at least two orders of magnitude.

7. The joining element according to one of the claims 1 to 6, **characterized in that** the joining element slowly contracts or tensions when affected below a threshold tension, but is substantially rigid when subjected to a shortlasting tensile load above the threshold tension.

8. The joining element according to claim 1, **characterized in that** the joining element is a thread material, which is constructed from several interwoven swellable threads each in turn surrounded by a threaded braid.

9. The joining element (40, 160, 170, 180, 190, 200) according to one of the claims 1 to 8, **characterized in that** the first material consists of substantially axially oriented threads (164) and/or surface components (161) axially enclosing the second material and that the second material comprises a swelling material, in particular a hygroscopic material, or a hydrophobic material with a hygroscopic inclusion.

10. The joining element according to one of the claims 1 to 9, **characterized in that** the second material is surrounded with a semipermeable membrane, respectively with a selectively permeable membrane or with a osmosis membrane.

11. A prosthetic material consisting of a textile structure such as a thread, nonwoven, woven, braid, knitted fabric, embroidered fabric, mesh or the like, made up of a multiplicity of joining elements according to one of claims 1 through 10.

12. The use of the joining element according to one of the claims 1 to 10 as a textile structure, preferably as a tissue.

13. The joining element according to one of the claims 1 ot 10 in the form of a textile structure as a pouch for enclosure of organs.

14. The joining element according to one of the claims 1 ot 10 in the form of a textile structure as fascial gaps.

15. The joining element according to one of the claims 1 ot 10 in the form of a textile structure as bridging graft for tendons or fascia defects.

16. The joining element according to one of the claims 1 ot 10 in the form of a textile structure as closure of skin defects.

17. The joining element according to one of the claims 1 ot 10 in the form of a textile structure as a cuff for vessels.

18. The joining element according to one of the claims 1 ot 10 in the form of a textile structure as support stockings, burns coverings for scar correction.

19. Non medical use of the joining element according to one of the claims 1 to 10 as tension thread, for producing a tension mesh or a textile membrane for packaging or securing of goods.

**Revendications**

1. Élément de liaison (40, 160, 170, 180, 190, 200), notamment un matériau filaire à usage chirurgical, ledit élément de liaison étant constitué d'un premier matériau (42, 162, 163, 164, 181, 183) qui est sensiblement rigide lorsqu'il est sollicité sur des côtés distants l'un de l'autre avec une contrainte de traction relativement courte dans le temps, le premier matériau comportant une structure réticulaire, et d'un second matériau (41, 165/166, 175/176, 165/186) relié au premier matériau (42, 162, 163, 164, 181, 183), l'élément de liaison se contractant lentement sur une seconde période plus longue que la première période, **caractérisé en ce que** l'élément de liaison (40, 160, 170, 180, 190, 200) se contract par gonflement du second matériau (41) et par cisaillement résultant de ce fait du premier matériau (42) en liaison avec le second matériau (41), le premier matériau (40, 160, 170, 180, 190, 200) entourant le second matériau (41, 165/166, 175/176, 165/186).

2. Élément de liaison selon la revendication 1, **caractérisé en ce que** le second matériau est un noyau sélectionné dans le groupe suivant :

 - un noyau comportant un matériau polymère en forme de fil, tel qu'un élastomère thermoplastique ou un élastomère réticulé ou un gel dans lequel sont incorporés des cristaux de sel (47) ; ou
 - un noyau comportant un tuyau qui est rempli d'un matériau expansible ou d'une substance active du point de vue osmotique ; ou

- un noyau (161) qui est limité par une membrane élastique, plastique ou extensible radialement du point de vue géométrique, un gel ou une matrice (165) dans laquelle sont incorporées des substances (166) actives du point de vue osmotiques, particulaires ou dissoutes sous forme de petites bulles, étant en outre prévu dans le noyau (161).

3. Élément de liaison selon la revendication 2, **caractérisé en ce que** le tuyau est découpé à intervalles réguliers afin de former des chambres segmentées.

4. Élément de liaison selon la revendication 1, **caractérisé en ce que** le second matériau est entouré d'une membrane semi-perméable ou d'une membrane perméable sélectivement ou d'une membrane osmotique.

5. Élément de liaison selon l'une des revendications précédentes, **caractérisé en ce que** la contrainte de traction relativement plus courte dans le temps s'exerce et/ou se relâche sur une première durée de moins de 1 minute.

6. Élément de liaison selon l'une des revendications précédentes, **caractérisé en ce que** la première durée est inférieure à la seconde durée d'au moins deux ordres de grandeur.

7. Éléments de liaison selon l'une des revendications précédentes, **caractérisé en ce que** l'élément de liaison se contracte ou se retend lentement dans le temps lorsqu'il est sollicité au-dessous d'une tension de seuil, mais est sensiblement rigide avec une contrainte de traction de courte durée au-dessus de la tension de seuil.

8. Élément de liaison selon l'une des revendications précédentes, **caractérisé en ce que** l'élément de liaison est un matériau filaire qui a élaboré à partir de plusieurs fils aptes au gonflement qui sont entremêlés et qui sont entourés chacun d'un entrelacement de fils.

9. Élément de liaison (40, 160, 170, 180, 190, 200) selon la revendication 1, **caractérisé en ce que** le premier matériau est constitué de fils (164) sensiblement orientés axialement et/ou de composants de surface (161) entourant axialement le second matériau, et **en ce que** le second matériau comporte un matériau gonflant, notamment un matériau hygroscopique ou un matériau hydrophobe doté d'une phase dispersée hygroscopique.

10. Élément de liaison selon l'une des revendications 1 à 9, **caractérisé en ce que** le second matériau est entouré d'une membrane semi-perméable ou d'une membrane perméable sélectivement ou d'une membrane osmotique.

11. Matériau prosthétique constitué d'un produit textile tel qu'un fil, un non-tissé, un tissu, un entrelacs, un produit tricoté, un produit brodé, un filet ou similaire constitué d'un grand nombre d'éléments de liaison selon l'une des revendications 1 à 10.

12. Utilisation de l'élément de liaison selon l'une des revendications 1 à 10 comme produit textile, de préférence comme tissu.

13. Élément de liaison selon l'une des revendications 1 à 10, se présentant sous la forme d'un produit textile, utilisé pour une poche destinée à contenir des organes.

14. Élément de liaison selon l'une des revendications 1 à 10 se présentant sous la forme d'un produit textile, utilisé pour des lacunes aponévrotiques.

15. Élément de liaison selon l'une des revendications 1 à 10, se présentant sous la forme d'un produit textile, utilisé pour l'interposition de tendons ou de défauts aponévrotiques.

16. Élément de liaison selon l'une des revendications 1 à 10 se présentant sous la forme d'un produit textile, utilisé pour l'obturateur de défauts cutanés.

17. Élément de liaison selon l'une des revendications 1 à 10 se présentant sous la forme d'un produit textile, utilisé pour des manchettes pour vaisseaux.

18. Élément de liaison selon l'une des revendications 1 à 10 se présentant sous la forme d'un produit textile, utilisé pour des bas de contention, un vêtement anti-brûlure, la correction de cicatrices.

19. Utilisation non médicale de l'élément de liaison selon l'une des revendications 1 à 10 comme fil tendeur pour la fabrication d'un filet tendeur ou d'une membrane textile pour l'emballage ou la protection de marchandises.

FIG. 1

FIG. 2

FIG. 3

FIG. 4

**FIG. 5**

**FIG. 6**

**FIG. 7**

**FIG. 7A**     **FIG. 8**

**FIG. 9**

**FIG. 10**

**FIG. 11**

**FIG. 12**

160

162
161
165
164
163
166

**FIG. 13**

170

162
161
171
177
175
164
163
176

**FIG. 14**

FIG. 15

FIG. 16

FIG. 17

FIG. 18

FIG. 19

26

FIG. 20

FIG. 21

**FIG. 22**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1284756 A

- US 6174333 B

**In der Beschreibung aufgeführte Nicht-Patentliteratur**

- **Garvin et al.** *Tissue Engineering,* 2003, vol. 9 (5), 967-979